# EUROPÄISCHE PATENTSCHRIFT

(11) **EP 0 509 042 B1**
(45) Veröffentlichungstag und Bekanntmachung des Hinweises auf die Patenterteilung: **02.03.1994**
(21) Anmeldenummer: 91902674.0
(22) Anmeldetag: 21.12.1990
(51) Int. Cl.: A61K 9/00, A61K 47/24, A61K 47/00, A61K 31/685

(54) **Intravenös applizierbare Pharmazeutische Zubereitung von 1-Octadecyl-2-methyl-glycero3-phosphocholin**
Pharmaceutical preparation of 1-Octadecyl-2-methyl-glycero-3-phosphocholin for intravenous administration
Composition pharmaceutique d'1-Octadecyl-2-methyl-glycero-3-phosphocholin administrable par voie intraveineuse

(30) Priorität: 03.01.1990 DE 4000084
(43) Veröffentlichungstag der Anmeldung: 21.10.1992
(73) Patentinhaber: MEDMARK PHARMA GMBH, 82031 Grünwald (DE)
(72) Erfinder: WIEBECKE, Dieter, D-8700 Würzburg (DE); NORDSTRÖM, Rabbe, D-8022 Grünwald (DE)
(74) Vertreter: Huber, Bernhard, Dipl.-Chem.
(86) Internationale Anmeldenummer: EP9002278
(87) Internationale Veröffentlichungsnummer: WO9109590

(56) Entgegenhaltungen:
- International Information Services, Datenbank: Chemical Abstracts, Access number: 104(6): 39731e, & JP, A, 60166610(FUJISAWA) 29 August 1985
- International Information Services, Datenbank: Chemical Abstracts, Access number: 98(4): 22276v, & JP, A, 57145811 (TOYAMA) 9 September 1982
- International Information Services, Datenbank: Chemical Abstracts, Access number: 110(23): 205219q, S. Mende et al.: "Effect of ET-18-OCH3/cholesterol liposomes on the membrane potential of isolated endothelial cells, & Pharmazie

## Beschreibung

Die Erfindung betrifft eine pharmazeutische Zubereitung zur intravenösen Applikation von ET18-OCH₃.

Es ist bekannt, daß Alkyllysophospholipide, welches synthetische Analoga der natürlich vorkommenden 2-Lysophosphatidylcholine sind, sowohl eine prophylaktische als auch eine therapeutische Wirksamkeit gegen das Wachstum von verschiedenen allogenischen und syngenischen Tumorarten aufweisen. Darüberhinaus ist auch die Inhibierung der Entwicklung von Metastasen beim Lungenkarzinom nachgewiesen. Die Antitumorwirkung dieser Alkyllysophospholipide wird durch zytotoxische Makrophagen und durch die direkte selektive Zerstörung von Neoplasmazellen bewirkt. Von den Alkyllysophospholipiden haben sich besonders das 1-Octadecyl-glycero-3-phosphocholin (ET18-OCH) und das 1-Octadecyl-2-methyl-glycero-3-phosphocholin (ET18-OCH₃) als besonders wirksam erwiesen.

Bislang war es jedoch nur möglich, ET18-OCH₃ am Zielorgan direkt oder auf oralem Wege in höheren Wirkdosen zu applizieren. W. Berdel et al., Anticancer Research I: 345-352 (1981) haben gefunden, daß eine intravenöse Verabreichung von ET18-OCH₃ nur bis zu einer Dosis von 40 mg/kg Körpergewicht bei Ratten und Mäusen verträglich ist, wohingegen schon bei 60 mg/kg die LD⁵⁰-Dosis erreicht wird. Dies trifft auch auf die Untersuchungen bei subhumanen Primaten zu. In allen Fällen konnte gezeigt werden, daß bei der intravenösen Verabreichung von ET18-OCH₃ eine Hämolyse stattfindet und daß für eine länger andauernde Behandlung lediglich geringe Dosen verabreicht werden können. Bei allen höheren Dosen traten bei den subhumanen Primaten Nebenwirkungen wie Durchfall, Erbrechen und vor allem Hämolyse auf, bei gleichzeitigem Anstieg der Bilirubin- und Harnstoffwerte im Blut sowie einer Erhöhung der Hämoglobin-, Protein- und Glucosewerte im Urin. Es ist bereits bekannt, daß sich diese Schwelle, bei der Nebenwirkungen auftreten, in Primaten durch den Zusatz von 20%igem Humanalbumin erhöhen läßt. Bei Humanversuchen wurde gefunden, daß sich bei einer täglichen Dosis von an Humanalbumin gebundenen 10 mg/kg ET18-OCH₃ über drei Wochen keine toxische Nebenwirkung nachweisen läßt. Jedoch tritt schon bei der Erhöhung der Dosis auf etwa 20 mg/kg eine Toxizität auf, die sich in einer Erhöhung des Serumkreatinins sowie in einer Glucosurie und Proteinurie und einer Verschlechterung der Leberwerte (Abfall der Serum-Cholinesterase) nachweisen läßt. Bei allen Dosishöhen bei der Verabreichung von ET18OCH3 zusammen mit Humanalbumin liegt ein Risiko für die Entstehung von Lungenödemen vor. Diese Tendenz ist umso ausgeprägter, je höher die Dosis ist. Die Erfindung hat nun zum Ziel, eine pharmazeutische Zubereitung von ET18-OCH₃ bereitzustellen, die sich intravenös verabreichen läßt und die die zuvor geschilderten Nachteile nicht aufweist.

Diese Aufgabe wird nun erfindungsgemäß gelöst durch eine pharmazeutische Zubereitung, die ET18-OCH₃ und eine lipophile, pharmazeutisch verträgliche Emulsion enthält.

Es wurde nämlich überraschenderweise gefunden, daß sich die Verträglichkeit von ET18-OCH₃ um ein Vielfaches erhöhen läßt, wenn der Wirkstoff mit einer solchen Emulsion der eingangs geschilderten Art verabreicht wird. Obwohl die gemeinsame Verabreichung von Emulsion und Wirkstoff bevorzugt ist hat es sich gezeigt, daß auch eine getrennte oder zeitlich abgestufte Verabreichung von Wirkstoff und Emulsion die therapeutische Verträglichkeit von ET18-OCH₃ erhöht.

Besonders überraschend ist es, daß eine Verabreichung von Fettemulsionen und ET18-OCH₃ überhaupt möglich ist, da bis zum Zeitpunkt der Erfindung bei der Verabreichung von Fettemulsionen die Zugabe von Arzneimittel-Wirkstoffen als kontraindiziert gegolten hat. Darauf wird auch bei den Gebrauchsinformationen der Hersteller solcher Emulsionen zur intravenösen Fettzufuhr hingewiesen (Gebrauchsinformation der B. Braun Melsungen AG D-3508 Melsungen, Bundesrepublik Deutschland, Fachinformation zu Lipofundin MCT und Fresenius AG, Bad Homburg V.D.H., 6370 Oberursel, Bundesrepublik Deutschland, Fachinformation zu Lipovenös).

Die in der erfindungsgemäßen Arzneimittel-Zubereitung vorliegende Emulsion enthält pharmazeutisch verträgliche lipophile Stoffe, die als feine Teilchen in einer wäßrigen Phase suspendiert vorliegen. Dabei weisen die Teilchen vorzugsweise eine Größenverteilung auf, wie sie in den natürlich vorkommenden Chylomikronen auftritt. Der bevorzugte durchschnittliche Teilchendurchmesser beträgt, wenn er mit der dem Fachmann bekannten dynamischen Lichtstreuung ermittelt wird, zwischen 200 und 800 nm und liegt vorzugsweise zwischen 300 und 500 nm. Besonders bevorzugt sind dabei Fettpartikel mit einem mittleren Teilchendurchmesser von 400 nm.

Zweckmäßigerweise enthält die lipophile Emulsion Öle, die vorzugsweise biologisch abbaubar sind. Als besonders zweckmäßig haben sich hierbei Öle erwiesen, wie sie aus Sojabohnen erhältlich sind.

Die in der erfindungsgemäßen Arzneimittel-Zubereitung enthaltene Emulsion weist in einer bevorzugten Ausführungsform einen Emulgator auf. Dabei handelt es sich vorzugsweise um biologisch metabolisierbare Emulgatoren, die hydrophile Gruppen wie ,-COONa, -SO₃Na, -O[C₂H₄O]NH und eine lipophile Gruppe wie z.B. einen Kohlenwasserstoffrest aufweisen. Üblicherweise werden als Emulgatoren jedoch Phosphorsäureester verwendet, worunter besonders Phospholipide und Phosphatidylcholin, insbesondere das 3-Sn-phosphatidylcholin, bevorzugt sind. Als besonders geeignet haben sich in der erfindungsgemäßen Emulsion auch Lecithine, insbesondere Eilecithine erwiesen. In einer bevorzugten Ausführungsform enthält die Emulsionslösung auch Triglyceride, insbesondere mittelkettige Triglyceride und/oder Glycerin. Zur Verbesserung der Ernährungslage bei geschwächten Patienten enthält die pharmazeutische Zubereitung auch einen Anteil an essentiellen Linol- und Linolensäure.

Das erfindungsgemäße Arzneimittel ist sowohl als fertige infundierbare Mischung als auch in einer Form lagerbar, in der das Pharmakon und die Emulsion getrennt vorliegen und erst bei ihrer Verwendung zur gleichzeitigen Verabreichung gemischt werden. Es hat sich jedoch auch gezeigt, daß mit der erfindungsgemäßen Arzneimittel-Zubereitung das Pharmakon und die Emulsion als Kombinationspräparat bei getrennter oder in zeitlich abgestufter Verabreichung die überraschende Wirkung aufweist. Die Erfindung wird durch die folgenden Beispiele näher erläutert.

### Beispiel 1

I. ET18-OCH₃ wurde nach dem Verfahren von Eibl, wie in der DE-OS 36 41 379 beschrieben, hergestellt. Dazu wurden 2705 g Octadecanol, 1317 g Mesylchlorid bei 30°C in 10 l trockenem THF gelöst und in einem Reaktor vorgelegt. Dazu wurden 1300 g Triethylamin in 2,5 l trockenem THF gelöst und unter starkem Rühren so zugetropft, daß die Reaktionstemperatur 40°C nicht übersteigt (30 Minuten weiterrühren). Die nach dem Zufügen von 10 l einer 2 n Salzsäure entstandene wäßrige Phase wird abgetrennt und verworfen. Nach dem Trocknen der organischen Phase über Natriumsulfat werden 2790 g Octadecylmesylat erhalten.
II. rac-1-Allyl-3-tert.-butyl-glycerin
   870 g Allylalkohol werden mit 14 g Natriumhydroxid unter Rückfluß gekocht, wobei das Natriumhydroxid in Lösung geht. In die kochende Lösung werden 781 g tert.-Butylglycidether langsam zugetropft und eine Stunde weitergekocht. Nach dem Extrahieren der Reaktionsmischung mit 1 l Wasser wird die wäßrige Phase zweimal mit je 300 ml Chloroform extrahiert und die vereinigten organischen Phasen unter Rückgewinnung des überschüssigen Allylalkohols destilliert. Auf diese Weise werden 910 g rac-1-Allyl-3-tert.-butyl-glycerin erhalten.
III. rac-1-Allyl-3-tert.-butyl-2-methyl-glycerin
   673 g Kalium-tert.-butylat werden in 1 l Dimethylformamid gelöst und es werden 940 g 1-Allyl-3-tert.-butyl-glycerin unter Rühren langsam zugetropft und anschließend auf 115 bis 120° erhitzt. Nach Beendigung der Reaktion wird die Reaktionsmischung auf 15° gekühlt und 694 g Dimethylsulfat in 500 ml Dimethylformamid so zugetropft, daß die Temperatur 20° nicht übersteigt. Nach beendeter Methylierung wird das Reaktionsgemisch mit 1 l Methanol und 2 l Diisopropylether versetzt und danach mit 2 l Wasser extrahiert. Die organische Phase wird abgetrennt und das Lösungsmittel abgezogen. Auf diese Weise werden 719 g dieses Zwischenproduktes erhalten.
IV. rac-1-tert.-Butyl-2-methyl-glycerin
   719 g des Zwischenproduktes III werden in 2,5 l Methanol, die 12 g Schwefelsäure enthalten, aufgekocht. Nach vollständiger Spaltung werden nach Abkühlung der Reaktionsmischung 10 g Calciumoxid eingerührt und man läßt über Nacht stehen, filtriert und zieht das Lösungsmittel ab. Nach einer Vakuumdestillation (1 mbar, 65°C) werden 356,4 g des Zwischenproduktes IV erhalten.
V. rac-2-Methyl-1-octadecyl-glycerin
   269,3 g Kalium-tert.-butylat werden in 750 ml Dimethylformamid gelöst und mit 356,4 g Zwischenprodukt IV unter Rühren versetzt. In das Reaktionsgemisch werden 697 g Octadecylmesylat in 1 l p-Xylol so zugetropft, daß die Temperatur auf 100°C ansteigt. Nach beendeter Reaktion wird bei Raumtemperatur abgekühlt und in die Reaktionsmischung 1 l Wasser, das 50 g Ameisensäure enthält, eingerührt. Nach der Phasentrennung wird die wäßrige Phase abgetrennt und verworfen und die organische Phase von p-Xylol befreit. Der Rückstand wird in 800 ml Eisessig aufgenommen, der 2 ml 70%ige Perchlorsäure enthält. Anschließend wird auf 60°C erwärmt, wobei sich der tert.-Butylrest abspaltet. Nach dem Abziehen von Methanol wird der Rückstand in Hexan aufgenommen, mit leicht sauer extrahiertem Wasser extrahiert und die organische Phase über Natriumsulfat getrocknet. Das Zwischenprodukt V wird bei -20°C ausgefroren.
VI. Kephalin und ET18-OCH₃
   914 g Phosphorylchlorid werden bei 0°C in 1 l Tetrahydrofuran vorgelegt und 1793 g 1-Octadecyl-2-methylglycerin, 905 g Triethylamin in 6 l THF so zugetropft, daß 10°C nicht überschritten werden. 652 g Ethanolamin, 905 g Triethylamin werden mit THF gemischt und das Gemisch zügig zur Reaktionslösung gegeben. Nach der Zugabe von 850 ml 33%iger Salzsäure in 4 l Wasser wird die organische Phase abgetrennt und abrotiert, der Rückstand mit 2-Propanol und Methylenchlorid versetzt und dem Reaktionsgefäß wieder zugeführt. Dazu werden zur Neutralisation der restlichen Salzsäure 3764 g Kaliumcarbonat in Wasser in verschiedenen Konzentrationen gelöst und stufenweise zugegeben. Danach werden bei 10°C 2018 g DMSSO zugetropft, daß eine Temperatur von 20 bis 30° nicht überschritten wird. Nach einstündigem Rühren wird die organische Phase abgetrennt und das Lösungsmittel abgezogen, bis ein noch fließfähiges Öl zurückbleibt, das mit Toluol mehrmals trockengezogen wird. Nach dem Aufnehmen des Rückstands in 5 l Methylenchlorid wird von den Salzen abfiltriert, das Filtrat mit 5 l THF versetzt und an Kieselgel chromatographiert, wobei ET18-OCH₃ absorbiert wird. Nach dem Abtrennen der apolaren Nebenprodukte wird das Produkt mit Methanol eluiert, das Lösungsmittel abgezogen und mit Methylenchlorid aufgenommen. Anschließend wird das Produkt unter Rühren mit Aceton gefällt, abfiltriert und mit Aceton gewaschen. Ausbeute 1571 g (≙ 60 %) ET18-OCH₃.

### Beispiel 2

Das nach Beispiel 1 erhaltenen ET18-OCH₃ wurde gelöst und es wurden damit Infusionslösungen hergestellt. Dabei wurden 300, 600 und 900 mg des Wirkstoffes in Aqua bidest. gelöst und mit 100 ml Lipofundin® MCT 10 % und MCT 20 % (B. Braun Melsungen AG, 5308 Melsungen, Bundesrepublik Deutschland) oder mit Lipovenös® 10 % oder 20 % (Fresenius AG, Bad Homburg, Bundesrepublik Deutschland) gemischt und an Probanden verabreicht. Hierbei wurde überraschenderweise gefunden, daß die Wirkung-Dosis-Beziehung nicht die sonst für pharmazeutische Wirkstoffe übliche S-förmig ansteigende Kurve ergab, sondern daß die Dosis/Wirkung von einer bestimmten Konzentration abhängig ist, die sich auch durch eine höhere Dosierung nicht weiter verändert. Dabei wurde auch gefunden, daß bei den meisten Probanden, unabhängig vom Körpergewicht, eine Tagesdosis von 300 mg über dieser kritischen Wirkungsgrenze liegt. Bei keinem der behandelten Patienten wurden die eingangs geschilderten Nebenwirkungen gefunden und zwar auch nicht bei Patienten, die mit solch hohen Dosen wie 900 mg behandelt wurden.

## Patentansprüche (Patentansprüche für folgende(n) Vertragsstaat(en): AT, BE, CH, DE, DK, ES, FR, GB, GR, IT, LI, LU, NL, SE)

1. Intravenös applizierbare pharmazeutische Zubereitung von 1-Octadecyl-2-methyl-glycero-3-phosphocholin, **dadurch** **gekennzeichnet**, daß sie eine lipophile Emulsion von Öl-in-Wasser enthält.

2. Pharmazeutische Zubereitung nach Anspruch 1, **dadurch** **gekennzeichnet**, daß die lipophile Emulsion metabolisierbare Öle enthält.

3. Pharmazeutische Zubereitung nach einem der vorhergehenden Ansprüche, **dadurch** **gekennzeichnet**, daß sie aus Sojabohnen erhältliche Öle enthält.

4. Pharmazeutische Zubereitung nach einem der vorhergehenden Ansprüche, **dadurch** **gekennzeichnet**, daß sie einen Emulgator enthält.

5. Pharmazeutische Zubereitung nach einem der vorhergehenden Ansprüche, **dadurch** **gekennzeichnet**, daß sie einen pharmazeutisch verträglichen Phosphosäureester enthält.

6. Pharmazeutische Zubereitung nach einem der vorhergehenden Ansprüche, **dadurch** **gekennzeichnet**, daß sie Phospholipide enthält.

7. Pharmazeutische Zubereitung nach einem der vorhergehenden Ansprüche, **dadurch** **gekennzeichnet**, daß sie Lecithine enthält.

8. Pharmazeutische Zubereitung nach einem der vorhergehenden Ansprüche, **dadurch** **gekennzeichnet**, daß sie Phosphatidylcholin enthält.

9. Pharmazeutische Zubereitung nach einem der vorhergehenden Ansprüche, **dadurch** **gekennzeichnet**, daß sie Triglyceride und/oder Glycerin enthält.

10. Pharmazeutische Zubereitung nach einem der vorhergehenden Ansprüche, **dadurch** **gekennzeichnet**, daß die in der Emulsion enthaltenen lipophilen Teilchen die Größe von Chylomikronen aufweisen.

11. Pharmazeutische Zubereitung nach einem der vorhergehenden Ansprüche, **dadurch** **gekennzeichnet**, daß die lipophilen Teilchen einen mittleren Teilchen-Durchmesser von 300 bis 500 nm aufweisen.

12. Pharmazeutische Zubereitung nach einem der vorhergehenden Ansprüche, **dadurch** **gekennzeichnet**, daß sie 1-Octadecyl-2-methyl-glycero-3-phosphocholin und die lipophile Emulsion als Kombinations-Präparat zur gleichzeitigen, getrennten oder zeitlich abgestuften Anwendung enthält.

13. Verwendung von 1-Octadecyl-2-methyl-glycero-3-phosphocholin und einer lipophilen Emulsion nach den Ansprüchen 1 bis 12 zur Herstellung eines Arzneimittels gegen multiple Sklerose, gegen Autoimmun-Erkrankungen, mit antiproliferativer Wirkung, zur Unterdrückung immunologischer Reaktionen, und zur Behandlung von Tumoren.

## Patentansprüche (Patentansprüche für folgende(n) Vertragsstaat(en): ES, GR)

1. Verfahren zur Herstellung einer intravenös applizierbaren pharmazeutischen Zubereitung von 1-Octadecyl-2-methyl-glycero-3-phosphocholin als Wirkstoff, **dadurch** **gekennzeichnet**, daß man den Wirkstoff in einer lipophilen Emulsion von Öl-in-Wasser auflöst.

2. Verfahren nach Anspruch 1, **dadurch** **gekennzeichnet**, daß die lipophile Emulsion metabolisierbare Öle enthält.

3. Verfahren nach einem der vorhergehenden Ansprüche, **dadurch** **gekennzeichnet**, daß die Emulsion aus Sojabohnen erhältliche Öle enthält.

4. Verfahren nach einem der vorhergehenden Ansprüche, **dadurch** **gekennzeichnet**, daß die Emulsion einen Emulgator enthält.

5. Verfahren nach einem der vorhergehenden Ansprüche, **dadurch** **gekennzeichnet**, daß die Emulsion einen pharmazeutisch verträglichen Phosphosäureester enthält.

6. Verfahren nach einem der vorhergehenden Ansprüche, **dadurch** **gekennzeichnet**, daß die Emulsion Phospholipide enthält.

7. Verfahren nach einem der vorhergehenden Ansprüche, **dadurch** **gekennzeichnet**, daß die Emulsion Lecithine enthält.

8. Verfahren nach einem der vorhergehenden Ansprüche, **dadurch** **gekennzeichnet**, daß die Emulsion Phosphatidylcholin enthält.

9. Verfahren nach einem der vorhergehenden Ansprüche, **dadurch** **gekennzeichnet**, daß die Emulsion Triglyceride und/oder Glycerin enthält.

10. Verfahren nach einem der vorhergehenden Ansprüche, **dadurch** **gekennzeichnet**, daß die in der Emulsion enthaltenen lipophilen Teilchen die Größe von Chylomikronen aufweisen.

11. Verfahren nach einem der vorhergehenden Ansprüche, **dadurch** **gekennzeichnet**, daß die lipophilen Teilchen einen mittleren Teilchen-Durchmesser von 300 bis 500 nm aufweisen.

12. Verwendung von 1-Octadecyl-2-methyl-glycero-3-phosphocholin und einer lipophilen Emulsion nach den Ansprüchen 1 bis 11 zur Herstellung eines Arzneimittels gegen multiple Sklerose, gegen Autoimmun-Erkrankungen, mit antiproliferativer Wirkung, zur Unterdrückung immunologischer Reaktionen, und zur Behandlung von Tumoren.

## Claims (Claims for the following Contracting State(s): AT, BE, CH, DE, DK, ES, FR, GB, GR, IT, LI, LU, NL, SE)

1. Intravenously administerable pharmaceutical composition of 1-octadecyl-2-methylglycero-3-phosphocholine, characterised in that it contains a lipophilic emulsion of oil-in-water.

2. Pharmaceutical composition according to claim 1, characterised in that the lipophilic emulsion contains metabolisable oils.

3. Pharmaceutical composition according to one of the preceding claims, characterised in that it contains oils obtainable from soya beans.

4. Pharmaceutical composition according to one of the preceding claims, characterised in that it contains an emulsifier.

5. Pharmaceutical composition according to one of the preceding claims, characterised in that it contains a pharmaceutically compatible phosphoric acid ester.

6. Pharmaceutical composition according to one of the preceding claims, characterised in that it contains phospholipids.

7. Pharmaceutical composition according to one of the preceding claims, characterised in that it contains lecithins.

8. Pharmaceutical composition according to one of the preceding claims, characterised in that it contains phosphatidylcholine.

9. Pharmaceutical composition according to one of the preceding claims, characterised in that it contains triglycerides and/or glycerol.

10. Pharmaceutical composition according to one of the preceding claims, characterised in that the lipophilic particles contained in the emulsion have the size of chylomicrons.

11. Pharmaceutical composition according to one of the preceding claims, characterised in that the lipophilic particles have an average particle diameter of 300 to 500 nm.

12. Pharmaceutical composition according to one of the preceding claims, characterised in that it contains 1-octadecyl-2-methylglycero-3-phosphocholine and the lipophilic emulsion as combination preparation for the simultaneous, separate or chronologically graded use.

13. Use of 1-octadecyl-2-methylglycero-3-phosphocholine and of a lipophilic emulsion according to claims 1 to 12 for the preparation of a medicament against multiple sclerosis, against auto-immune diseases, with anti-proliferative action, for the suppression of immunological reactions and for the treatment of tumours.

## Claims (Claims for the following Contracting State(s): ES, GR)

1. Process for the preparation of an intravenously administerable pharmaceutical composition of 1-octadecyl-2-methylglycero-3-phosphocholine as active material, characterised in that one dissolves the active material in a lipophilic emulsion of oil-in-water.

2. Process according to claim 1, characterised in that the lipophilic emulsion contains metabolisable oils.

3. Process according to one of the preceding claims, characterised in that the emulsion contains oils obtainable from soya beans.

4. Process according to one of the preceding claims, characterised in that the emulsion contains an emulsifier.

5. Process according to one of the preceding claims, characterised in that the emulsion contains a pharmaceutically compatible phosphoric acid ester.

6. Process according to one of the preceding claims, characterised in that the emulsion contains phospholipids.

7. Process according to one of the preceding claims, characterised in that the emulsion contains lecithins.

8. Process according to one of the preceding claims, characterised in that the emulsion contains phosphatidylcholine.

9. Process according to one of the preceding claims, characterised in that the emulsion contains triglycerides and/or glycerol.

10. Process according to one of the preceding claims, characterised in that the lipophilic particles contained in the emulsion have the size of chylomicrons.

11. Process according to one of the preceding claims, characterised in that the lipophilic particles have an average particle diameter of 300 to 500 nm.

12. Use of 1-octadecyl-2-methylglycero-3-phosphocholine and of a lipophilic emulsion according to claims 1 to 11 for the preparation of a medicament against multiple sclerosis, against auto-immune diseases, with anti-proliferative action, for the suppression of immunological reactions and for the treatment of tumours.

## Revendications (Revendications pour l'(les) Etat(s) contractant(s) suivant(s): AT, BE, CH, DE, DK, ES, FR, GB, GR, IT, LI, LU, NL, SE)

1. Préparation pharmaceutique d'1-octadécyl-2-méthyl-glycéro-3-phosphocholine applicable par voie intraveineuse caractérisée en ce qu'elle contient une émulsion lipophile d'huile dans l'eau.

2. Préparation pharmaceutique selon la revendication 1, caractérisée en ce que l'émulsion lipophile contient des huiles métabolisables.

3. Préparation pharmaceutique selon l'une des revendications précédentes, caractérisée en ce qu'elle contient des huiles qui peuvent être obtenues à partir de graines de soja.

4. Préparation pharmaceutique selon l'une des revendications précédentes, caractérisée en ce qu'elle contient un émulsifiant.

5. Préparation pharmaceutique selon l'une des revendications précédentes, caractérisée en ce qu'elle contient un ester phosphorique acceptable du point de vue pharmaceutique.

6. Préparation pharmaceutique selon l'une des revendications précédentes, caractérisée en ce qu'elle contient des phospholipides.

7. Préparation pharmaceutique selon l'une des revendications précédentes, caractérisée en ce qu'elle contient des lécithines.

8. Préparation pharmaceutique selon l'une des revendications précédentes, caractérisée en ce qu'elle contient de la phosphatidylcholine.

9. Préparation pharmaceutique selon l'une des revendications précédentes, caractérisée en ce qu'elle contient des triglycérides et/ou du glycérol.

10. Préparation pharmaceutique selon l'une des revendications précédentes, caractérisée en ce que les particules lipophiles contenues dans l'émulsion présentent la taille de chylomicrons.

11. Préparation pharmaceutique selon l'une des revendications précédentes, caractérisée en ce que les particules lipophiles présentent un diamètre moyen de 300 à 500 nm.

12. Préparation pharmaceutique selon l'une des revendications précédentes, caractérisée en ce qu'elle contient de l'1-octadécyl-2-méthyl-glycéro-3-phosphocholine et l'émulsion lipophile sous forme d'une préparation par combinaison pour une utilisation simultanée, séparée ou échelonnée dans le temps.

13. Utilisation de l'1-octadécyl-2-méthyl-glycéro-3-phosphocholine et d'une émulsion lipophile selon les revendications 1 à 12 pour la préparation d'un médicament contre la sclérose en plaques, contre les maladies auto-immunes, à action antiproliférative, pour réprimer les réactions immunologiques et pour le traitement des tumeurs.

## Revendications (Revendications pour l'(les) Etat(s) contractant(s) suivant(s): ES, GR)

1. Procédé d'obtention d'une préparation pharmaceutique applicable par voie intraveineuse dont le principe actif est l'1-octadécyl-2-méthyl-glycéro-3-phosphocholine, caractérisé en ce que l'on dissout le principe actif dans une émulsion lipophile d'huile dans l'eau.

2. Procédé selon la revendication 1, caractérisé en ce que l'émulsion lipophile contient des huiles métabolisables.

3. Procédé selon l'une des revendications précédentes, caractérisé en ce que l'émulsion contient des huiles qui peuvent être obtenues à partir de graines de soja.

4. Procédé selon l'une des revendications précédentes, caractérisé en ce que l'émulsion contient un émulsifiant.

5. Procédé selon l'une des revendications précédentes, caractérisé en ce que l'émulsion contient un ester phosphorique acceptable du point de vue pharmaceutique.

6. Procédé selon l'une des revendications précédentes, caractérisé en ce que l'émulsion contient des phospholipides.

7. Procédé selon l'une des revendications précédentes, caractérisé en ce que l'émulsion contient des lécithines.

8. Procédé selon l'une des revendications précédentes, caractérisé en ce que l'émulsion contient de la phosphatidylcholine.

9. Procédé selon l'une des revendications précédentes, caractérisé en ce que l'émulsion contient des triglycérides et/ou du glycérol.

10. Procédé selon l'une des revendications précédentes, caractérisé en ce que les particules lipophiles contenues dans l'émulsion présentent la taille de chylomicrons.

11. Procédé selon l'une des revendications précédentes, caractérisé en ce que les particules lipophiles présentent un diamètre moyen de 300 à 500 nm.

12. Utilisation de l'1-octadécyl-2-méthyl-glycéro-3-phosphocholine et d'une émulsion lipophile selon les revendications 1 à 11 pour la préparation d'un médicament contre la sclérose en plaques, contre les maladies auto-immunes, à action antiproliférative, pour réprimer les réactions immunologiques et pour le traitement des tumeurs.
